# EUROPÄISCHE PATENTANMELDUNG

(11) **EP 2 985 021 A1**
(43) Veröffentlichungstag der Anmeldung: **17.02.2016**
(21) Anmeldenummer: 15178466.7
(22) Anmeldetag: 02.02.2009
(51) Int. Cl.: A61K 31/155, A61P 35/00, A61P 33/02, A61P 31/18, A61P 31/16, A61P 7/02, A61P 25/28, A61P 9/12, A61K 31/222, C07C 251/66

(54) **VERWENDUNG VON AMIDOXIMCARBONSÄUREESTERN UND N-HYDROXYGUANIDINCARBONSÄUREESTERN ZUR HERSTELLUNG VON PRODRUGS**

(30) Priorität: 01.02.2008 DE 102008007381
(62) Teilanmeldung aus: 09705303.7
(71) Anmelder: Dritte Patentportfolio Beteiligungsgesellschaft mbH & Co. KG, 12529 Schönefeld/OT Waltersdorf (DE)
(72) Erfinder: CLEMENT, Bernd, 24106 Kiel (DE); HUNGELING, Helen, 24116 Kiel (DE); Wolter-Reeh, Christiane, 25488 Holm (DE)
(74) Vertreter: Zech, Stefan Markus

(57) **Zusammenfassung**

Die vorliegende Erfindung betrifft ein Verfahren zur Verbesserung der Bioverfügbarkeit und Ermöglichung der Blut-Hirn-Schrankengängigkeit von Arzneistoffen, die wenigstens eine oder mehrere Amidin-, Guanidin-, *N*-Hydroxyamidin- (Amidoxim) bzw. *N*-Hydroxyguanidin-Funktionen aufweisen und entsprechend modifizierte Arzneistoffe enthaltende Arzneimittel.

## Beschreibung

Die vorliegende Erfindung betrifft eine Verwendung eines Amidoximcarbonsäureesters bei einem Verfahren zur Verbesserung der Bioverfügbarkeit und Ermöglichung der Blut-Him Schrankengängigkeit von Arzneistoffen, die wenigstens eine oder mehrere Amidin-, Guanidin-, *N*-Hydroxyamidin- (Amidoxim) bzw. *N-*Hydroxyguanidin-Funktionen aufweisen und entsprechend modifizierte Arzneistoffe enthaltende Arzneimittel.

*N*-Hydroxyamidine (Amidoxime) und *N-*Hydroxyguanidine stellen bekannte Prodrug-Prinzipien zur Erhöhung der oralen Bioverfügbarkeit der Amidine [Clement, B. Methoden zur Behandlung und Prophylaxe der Pneumocystis carinii Pneumonie (PCP) und anderen Erkrankungen sowie Verbindungen und Formulierungen zum Gebrauch bei besagten Methoden. P 432444.4, 1993] und Guanidine dar.

Pharmazeutische Zubereitungen, die einen Wirkstoff mit einer oder mehreren Amidin- oder Guanidin-Funktionen enthalten, zeigen bei oraler Anwendung nahezu keine pharmakologische Wirkung. Die Voraussetzung für einen therapeutischen Effekt eines Wirkstoffs nach oraler Gabe stellt dessen Aufnahme aus dem Gastrointestinaltrakt dar. Der wichtigste Mechanismus eines solchen Effekts ist die passive Diffusion. Der Grad der Resorption auf dem Weg der passiven Diffusion ist abhängig von der Lipophilie und somit auch abhängig von der Azidität und der Basizität des Wirkstoffs.

Stark basische Verbindungen, wie Amidine und Guanidine, liegen im Magen (pH 1) und im Dünndarm (pH 6,4) nahezu vollständig protoniert vor. Eine Resorption nach oraler Gabe, die den Durchtritt durch die Lipiddoppelschichten der Membranen des Gastrointestinaltrakts erfordert, erfolgt daher nur in sehr geringem Ausmaß.

Ein weiteres Problem bei der medikamentösen Behandlung vieler Krankheiten ist die Notwendigkeit die Blut-Hirn-Schranke zu passieren. Die Blut-Him-Schranke stellt eine effektive Barriere in Bezug auf die Resorption von Substanzen ins Gehirn dar. Sie sichert die selektive Aufnahme und verhindert das Eindringen von Substanzen. Darüber hinaus fungiert die Blut-Hirn-Schranke nicht nur als physikalische, sondern auch als enzymatische Barriere. An der Penetration von Substanzen ins Gehirn sind verschiedene Prozesse beteiligt Im Handel befinden sich im Vergleich zu anderen Indikationen nur wenige Arzneimittel, die im Zentralen Nervensystem (ZNS) ihre Wirkung entfalten. Davon gelangt der überwiegende Teil durch Diffusion ins ZNS. Auf diesem Wege werden Krankheiten wie die Epilepsie, chronische Schmerzen oder Depressionen behandelt. Andere schwerwiegende Funktionsstörungen wie beispielsweise Himtumore oder die Amyotrophe Lateralsklerose können auf diesem Wege heutzutage noch nicht behandelt werden [PARDRIDGE, W. M. NeuroRx 2005, 2, 3-14]. Um die Blut-Him-Schranke durch passive Diffusion überwinden zu können, muss eine Sustanz lipophil sein, ein geringeres Molekulargewicht besitzen als 400-500 Da, und sie muss ungeladen vorliegen. Um spezifisch kleine Moleküle, wie Glucose oder Aminosäuren zu resorbieren, sind verschiedene Transporter-Systeme, wie beispielsweise Nukleosid-Transporter, Influx- und Efflux-Transporter für organische Anionen, Glucose-Transporter, Peptid-Transporter und Aminosäure-Transporter an der Blut-Him-Schranke exprimiert [TAMAI, L; Tsun, A. J Pharm Sci 2000, 89, 1371-1388 UND DEBOER, A.; VAN DER SANDT, I. Annu Rev Pharmacol Toxicol 2003, 43, 629-656]. Größere Moleküle, wie Insulin oder eisenhaltiges Transferrin, werden über den rezeptorvermittelten Transport aufgenommen. Dabei spielen besonders der Insulin-und der Transferrinrezeptor eine große Rolle [DE BOER, A.; VAN DER SANDT, I. Annu Rev Pharmacol Toxicol 2003, 43, 629-656; PARDRIDGE, W. M. Mol Interv 2003, 3, 90-105]. Am Beispiel der Aminosäure L-Dopa hat man sich eines Prodrug-Prinzips bedient, damit das wasserlösliche Catecholamin Dopamin die Blut-Hirn-Schranke überwinden kann. Der Transport geschieht durch den Aminosäure-Transporter LAT1 (large neutral amino acid transporter). Nach der Passage erfolgt die Decarboxylierung zu Dopamin [PARDRIDGE, W. M. Mol Interv 2003, 3, 90-105].

Diamidine werden als Antiparasitika gegen Malaria, gegen die Pneumocystis jiroveci (früher carinii) Pneumonie, gegen die Trypanosomiasis (Afrikanische Schlafkrankheit) und gegen die Leishmaniose eingesetzt [WERBOVETZ, K. Curr Opin Investig Drugs 2006, 7, 147-157]. Insbesondere in den Entwicklungsländern stellen diese Erkrankungen ein ernstzunehmendes Problem mit hohen Sterblichkeitsraten dar.

Auf dem Markt befinden sich drei parenteral zu applizierende Diamidine. Pentamidin (Pentacarinat^{®}) wird schon seit 60 Jahren im Frühstadium der Afrikanischen Schlafkrankheit eingesetzt Im 2. Stadium der Afrikanischen Schlafkrankheit, dem meningoenzephalitischem Stadium liegt keine Wirksamkeit mehr vor, da die Blut-Him-Schranke nicht erfolgreich passiert werden kann. Es müssen daher stark toxische Arsen-Verbindungen gegeben werden. Es besteht ein Mangel an Arzneistoffen, die im 2. Stadium der Afrikanischen Schlafkrankheit wirken.

Es ist zu vermuten, dass alle Wirkstoffe, die als funktionelle Gruppe ein Amidin oder ein Guanidin aufweisen, eine ungenügende Resorption bei der oralen Anwendung zeigen, wenn sie nur durch passive Diffusion aufgenommen werden können.

Arzneistoffe mit Carbonsäuren sind sehr weit verbreitet und können auch als orale Arzneiformen angewendet werden. Hier sind besonders alle Analgetika aus der Gruppe der Essig-, Propion- und Salicylsäurederivate zu nennen.

Die *N*-hydroxylierten Derivate, wie die Amidoxime und die *N*-Hydroxyguanidine, zeigen durch die Einführung des Sauerstoffatoms eine geringere Basizität Unter physiologischen Bedingungen liegen sie nicht protoniert vor. Benzamidoxim stellt eine Modellverbindung für viele Arzneistoffe dar, die eine Amidoxim-Funktion enthalten [Clement, B. Drug Met Rev 2002, 34, 565-579].

Pentamidin und Diminazen stellen Diamidine dar und werden nach oraler Applikation nicht resorbiert. Daher wurden sie in Amidoxim-Prodrugs überführt. (DE 10 2006 034 256.9).

Am Beispiel des Pentamidins zeigt sich, dass mit der Überführung in den Pentoximester auch eine Verringerung der Löslichkeit einhergeht Dieses ist wahrscheinlich auch der Grund für die nicht hundertprozentige Bioverfügbarkeit des Pentamidins nach oraler Applikation des Pentoximesters [Clement B.; Bürenheide A; Rieckert W.; Schwarz J. ChemMedChem 2006, 1, 1260-7].

Des Weiteren führt die verminderte Wasserlöslichkeit zu dem Nachteil, dass eine Gabe des Arzneimittels durch Injektion wässriger Lösungen nicht mehr möglich ist. Dieses ist insbesondere dann ein Problem, wenn eine orale Gabe nicht in Frage kommt

Daher ist es die Aufgabe der vorliegenden Erfindung, die Wasserlöslichkeit und somit auch die orale Bioverfügbarkeit von Substanzen, die in Amidoxim- bzw. in N-Hydroxyguanidin-Prodrugs überführt wurden, zu erhöhen und / oder somit das Passieren der Blut-Him-Schranke zu ermöglichen.

Die Aufgabe wird durch die erfindungsgemäße Verwendung mit den Merkmalen nach Anspruch 1 gelöst Die Unteransprüche geben vorteilhafte Ausgestaltungen der Erfindung an.

### Beschreibung der Erfindung

Erfindungsgemäß wird vorgeschlagen, Amidoximcarbonsäureester (I) und der *N-*Hydroxyguanidincarbonsäureester (II) der Formeln: 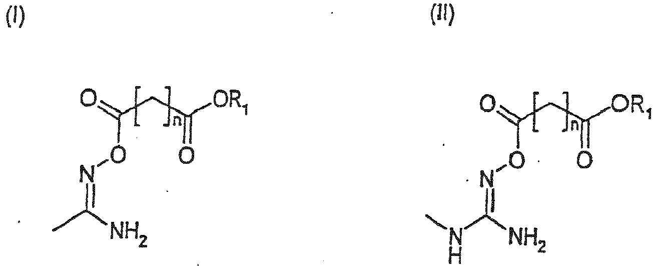 wobei für n gilt: n = 0 - 12 und R₁ Wasserstoff, einen Alkyl- oder einen Arylrest darstellt, oder deren Salze als Ersatz einer oder mehrerer Amidin-, *N*-Hydroxyamidin- (Amidoxim), Guanidin- bzw. *N*-Hydroxyguanidin-Funktionen eines Arzneistoffs in Arzneimitteln zur Verbesserung der Löslichkeit, Bioverfügbarkeit und Blut-Hirn-Schrankengängigkeit des Arzneistoffe zu verwenden.

Wird n größer als 12 gewählt, so ist eine Verbesserung der Löslichkeit, Bioverfügbarkeit und Blut-Hirn-Schrankengängigkeit nicht mehr gegeben.

*N-*Hydroxyamidine (Amidoxime) und *N*-Hydroxyguanidine sind erfolgreiche Prodrug-Prinzipien zur Erhöhung der oralen Bioverfügbarkeit der Amidine (DE 10 2006 034 256.9).

Die erfindungsgemäß vorgeschlagene Veresterung der Amidoxime bzw. der *N-*Hydroxyguanidine mit mit Dicarbonsäuren und Estern der Dicarbonsäuren verbessert die Löslichkeit, insbesondere die Löslichkeit in wässrigen Medien und die Bioverfügbarkeit gegenüber den bekannten Prodrugs erheblich.

Die Veresterung der Amidoxime bzw. der *N*-Hydroxyguanidine mit Dicarbonsäuren/Dicarbonsäureestem stellt aufgrund der eingeführten negativen Ladung der deprotonierten Säurefunktion eine Umpolung der Ladungen im Vergleich zum Benzamidin dar, somit wird eine erhöhte Bioverfügbarkeit und Löslichkeit in wässrigen Medien bewirkt

Der besondere Vorteil der erfindungsgemäßen Verbindungen liegt darin, dass sie im Blut deprotoniert vorliegen und so als Substrat für den Transporter für organische Anionen (organic anion transporter (OAT)) wirken und auf diesem Wege eine deutliche verbesserte Absorption aus dem Gastrointestinaltrakt und damit erhöhte Bioverfügbarkeit zeigen, die Überwindung der Blut-Him-Schranke durch derartige Transporter für organische Anionen kann somit ermöglicht werden. Dies wäre ein entscheidender Fortschritt in der Therapie der Afrikanischen Schlafkrankheit, da auch die 2. Phase der Erkrankung effektiv behandelt werden kann.

Darüber hinaus soll diese Veresterung die Überwindung der Blut-Him-Schranke ermöglichen. Im Blut wird die Verbindung deprotoniert vorliegen, so dass sie ein Substrat für den Transporter für organische Anionen (organic anion transporter (OAT)) an der Blut-Hirn-Schranke darstellen könnte.

Durch die Einführung von Carbonsäuren sind auch injizierbare Arzneiformen möglich, da wie bei den Amidinen die Wasserlöslichkeit wieder hergestellt wird. Dieses trifft insbesondere gut auf den Fall dass R₁ gleich Wasserstoff ist zu.

Gemäß der erfindungsgemäßen Verwendung wird durch das Ersetzen wenigstens einer oder mehrerer Amidin-, *N*-Hydroxyamidin- (Amidoxim), Guanidin- bzw. *N*-Hydroxyguanidin-Funktionen durch den Amidoximdicarbonsäureester und den *N-*Hydroxyguanidindicarbonsäureester erreicht, dass die Löslichkeit des betreffenden Arzneistoffs erhöht wird. Dadurch kann er nach oraler Verabreichung zunächst effektiv resorbiert und anschließend durch körpereigene Esterasen und *N*-Reduktasen wieder in die eigentliche Wirkform, das Amidin bzw. das Guanidin, zurückverwandelt werden (Prodrug-Prinzip). Die ausgezeichnete Resorbierbarkeit der abgewandelten Amidoxim- bzw. *N-*Hydroxyguanidin-Funktion im Gastrointestinaltrakt ist offensichtlich auf die erhöhte Löslichkeit der Wirkstoffmoleküle zurückzuführen. Darüber hinaus können die neuen Prodrug-Prinzipien die Überwindung der Blut-Him-Schranke ermöglichen.

Es ist ausreichend, wenn der Wirkstoff mindestens eine oder mehrere wirksame Amidin*-, N-*Hydroxyamidin- (Amidoxim), Guanidin- bzw. *N*-Hydroxyguanidin-Funktionen in der vorgeschlagenen Form enthält Der Wirkstoff kann demnach z.B. mehrere Amidoxim-Funktionen (z.B. zwei wie bei dem Pentoximester) bzw. *N*-Hydroxyguanidin-Funktionen enthalten, wobei dann mindestens eine dieser Gruppen in der vorstehend beschrieben Art modifiziert ist Genauso können auch Mischungen von Wirkstoffen eingesetzt werden, sofern mindestens ein Wirkstoff eine oder mehrere Amidin-, *N*-Hydroxyamidin- (Amidoxim), Guanidin- bzw. *N*-Hydroxyguanidin-Funktionen aufweist. Die orale Darreichungsform kann als flüssige, halbfeste oder feste Zubereitung, insbesondere als Tablette, Dragee, Pellet oder Mikrokapsel aufgemacht sein. Hierbei werden für solche Ausführungsformen, bei denen flüssige Zubereitungen eingesetzt werden, der Wirkstoff bzw. das Wirkstoffgemisch in einem geeigneten, nicht toxischen Lösungsmittel, wie zum Beispiel Wasser, einwertige Alkohole, insbesondere Ethanole, mehrwertige Alkohole, insbesondere Glycerin und/oder Propandiol, Polyglykole, insbesondere Polyethylenglykole und/oder Miglyol, Glycerinfomal, Dimethylisosorbit, natürliche oder synthetische Öle, aufgenommen. Für die Herstellung von halbfesten oder festen Zubereitungen gelangen die üblichen Grundmassen, wie beispielsweise Bentonit, Veegum, Guarmehl und/oder Cellulosederivate, insbesondere Methylcellulose und/oder Carboxymethylcellulose, sowie Polymere aus Vinylalkoholen und/oder Vinylpyrrolidonen, Alginate, Pektine, Polyacrylate, feste und/oder flüssige Polyethylenglykole, Paraffine, Fettalkohole, Vaseline und/oder Wachse, Fettsäuren und/oder Fettsäureester zur Anwendung.

Des Weiteren können in festen Zubereitungen die an sich bekannten Streckmittel, wie beispielsweise kolloidale Kieselsäure, Talkum, Milchzucker, Stärkepulver, Zucker, Gelatine, Metalloxide und/oder Metallsalze enthalten sein. Als weitere Zusatzstoffe bieten sich Stabilisatoren, Emulgatoren, Dispergatoren sowie Konservierungsstoffe an.

Die nach der erfindungsgemäßen Verwendung modifizierten Arzneistoffe weisen bei oraler Verabreichung eine hervorragende Resorbierbarkeit und somit Bioverfügbarkeit auf, wodurch die pharmakologische Wirkung des Amidins bzw. des Guanidins deutlich erhöht wird. Somit kann nunmehr eine optimale Arzneiform für die orale Anwendung von Amidinen bereitgestellt werden.

Besondere Bedeutung erlangt die erfindungsgemäße Verwendung dadurch, dass die funktionellen Gruppen Amidin und Guanidin wesentliche Bestandteile von verschiedenen wichtigen Wirkstoffen für verschiedene Anwendungsgebiete sind. Sie sind u.a. Bestandteil der folgenden Wirkstoffklassen bzw. Wirkstoffe: Proteasehemmstoffe (Thrombinhemmstoffe wie Melagatran, Hemmstoffe von Faktor Xa, Faktor VII bzw. aller Proteasen der Gerinnungskaskade; Matriptasehemmstoffe), Antikoagulantien, Thrombolytika, Antifibrinolytika, DNA- und RNA- interkalierende Verbindungen (wie Pentamidin, Diminazen, Isometamidium), N-Methyl-D-Aspartat Rezeptor-Antagonisten und Hemmstoffe viraler Enzyme (wie z. B. Neuraminidasehemmstoffe).

Wirkstoffe, die eine wirksame Amidin-Funktion bzw. Guanidin-Funktion enthalten, können u.a. zur Hemmung der Blutgerinnung, zur Prophylaxe und Therapie der viszeralen und der kutanen Leishmaniose, der Trypanosomiasis (Afrikanische Schlafkrankheit), der durch Pneumocystis carinii verursachten Pneumonie (PCP), zur Wachstumshemmung maligner Tumoren, Blutdrucksenkung, Neuroprotektion, sowie zur Bekämpfung von viralen Infektionen wie Influenza und von HIV-Infektionen eingesetzt werden.

Die obigen Aufzählungen sind nur beispielhaft und die Erfindung umfasst grundsätzlich alle Wirkstoffe, die mindestens eine Amidin-Funktion bzw. Guanidin-Funktion aufweisen, welche erfindungsgemäß in ein verbessertes Prodrug überführt wurde. Das erfindungsgemäße Verfahren ist somit auf einen sehr breiten Bereich von Wirkstoffklassen und Indikationen anwendbar und kann die Bioverfügbarkeit von vielen Arzneistoffen, deren aktive Form ein Amidin bzw. ein Guanidin enthält, deutlich erhöhen.

Als Beispiele für nach dem erfindungsgemäßen Verfahren modifizierte Verbindungen seien das *O*-Succinylbenzamidoxim, der Pentamidinbernsteinsäureester und der Diminazenbernsteinsäureester genannt.

Die erfindungsgemäße Zubereitung wird anhand von Ausführungsbeispielen näher erläutert:
Die erfindungsgemäße Zubereitung wird anhand von Ausführungsbeispielen im Kapitel Material und Methoden näher erläutert.

### Material und Methoden

### 1) Ausführungsbeispiel 1:

### O-Succinylbenzamidoxim

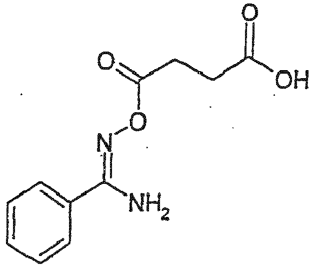

1,0 g Benzamidoxim wurden in 40 ml wasserfreiem Aceton gelöst Nach Zugabe von 1,64 g Bernsteinsäureanhydrid wurde vier Stunden unter Rückfluss gerührt. Die Vollständigkeit der Umsetzung wurde mittels DC überprüft. Nach beendeter Reaktion wurde das Lösungsmittel vollständig abgezogen und das Rohprodukt aus Toluol umkristallisiert.
Ausbeute: 82 %
Schmelzpunkt: 151°C
IR (KBr):
Y=3648, 3480, 3062, 2912, 1744, 1704, 1614, 1368 cm⁻¹.
¹H-NMR (300 MHz, DMSO-*d₆*):
δ/ppm (TMS) = 2,55 (t, 2H, *³J* = 6,9 Hz, CH₂), 2,69 (t, 2H, *³J* = 6,9 Hz, CH₂), 6,75 (s, 2H, NH₂), 7,44 (m, 3H, Ar-H), 7,70 (m, 2H, Ar-H), 12,20 (s, 1H, COOH).
¹³C-NMR (75 MHz, DM*SO*-*d₆*)*:*
δ/ppm (TMS) = 27,86 (CH₂), 28,72 (CH₂), 126,65 (C*_{Ar}* 2,6), 128,26 (C_{Ar} 3,5), 130,36 (C_{Ar} 1), 131,59 (C_{Ar} 4), 156,56 (C-NH₂), 170,19 (C=O), 173,50 (COOH).
MS (ESI):
*m*/*z* (%) = 495 [2M+Na+H⁺] (100), 259 [M+Na+H⁺] (20), 237 [M+H⁺] (43), 137 [BAO+H⁺] (35), 121 [BA+H⁺](13), 119 [C₄H₆O₄+H⁺] (70).
C₁₁H₁₂N₂O₄ (236,23):

| | | | |
|---|---|---|---|
| Ber. | C 55,93% | H 5, 12% | N 11,86% |
| Gef. | C 55,88% | H 5,19% | N 11,55% |

Zum Nachweis der Resorption ans dem Gastrointestinaltrakt and der anschließenden Re duktion zu Benzamidin wird das *O*-Succinylbenzamidoxim als Modellverbindungen für die neuen Prodrug-Prinzipien gewählt und jeweils drei Ratten oral verabreicht Die Metabolisierung der beiden Ester zu Benzamidin verläuft dabei *in vivo* folgendermaßen:

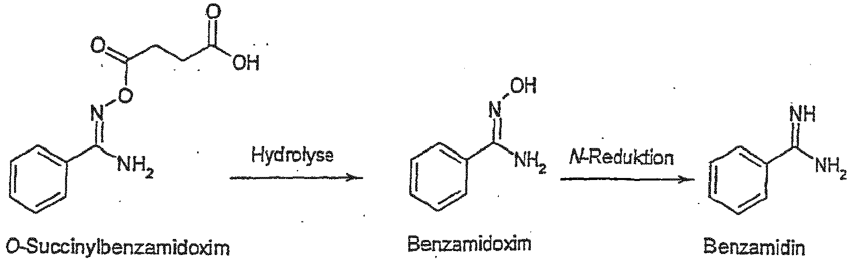

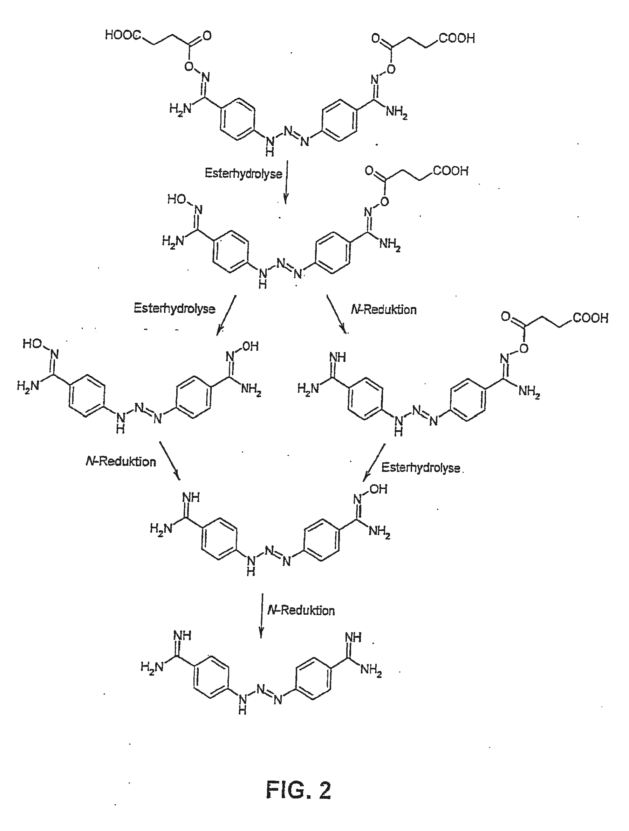

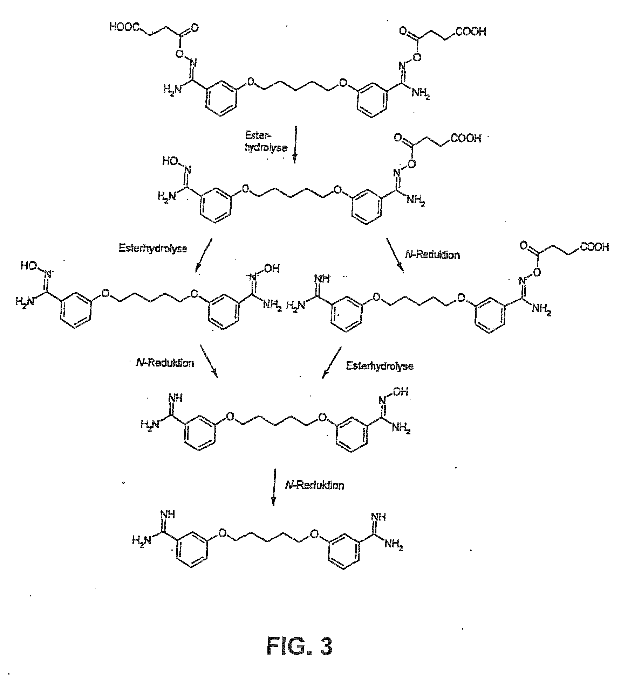

Der Metabolismus des Diminazenbemsteinsäureesters und des Pentamidinbernsteinsäureesters ist in den Fig. 2-3 im Anhang dargestellt.

### METHODEN BEZÜGLICH DER DURCHFÜHRUNG DER RATTENSTUDIE

Der Tierversuch wurde vom Ministerium für Landwirtschaft, Umwelt und längliche Räume des Landes Schleswig-Holstein am 04.07.2007 genehmigt.

Die Narkose erfolgt mittels Xylazin und Ketamin. Beides wurde i.m. injiziert. Die Silikonkatheter werden in die Vena jugularis und in die Arteria carotis implantiert Sie besitzen lokal antithrombotisch und antiinflammatorische Eigenschaften, wirken aber nicht systemisch. Die Augen wurden während der Operation mittels einer comeaprotektiven Salbe (Oculotect^{®}) geschützt sowie 3-4 ml Ringer-Lactat-Lösung s.c. zur Verbesserung der postoperativen Energieversorgung appliziert. Die Tiere werden antiphlogistisch (Finadyne^{®}, 1mg/kg KG) und antibiotisch (Amoxicillin^{®} 15%, 10 mg/kg KG) behandelt und postoperativ bis zum Aufwachen betreut und warm gehalten. Am Tag nach der Operation erhalten die Tiere Nutri plus^{®}, eine Energiepaste (Sojaöl, Melasse, Lebertranöl, Fleischextrakt, Mineralstoffvormischung, Vitaminvormischung).

Die Tiere werden nach Beendigung des Versuches mit Pentobarbital (i.v.) eufhanasiert.

### HALTUNG DER RATTEN

Als Versuchstiere dienen männliche Wistar-Ratten mit einem durchschnittlichen Gewicht von 200 g. Die Tiere werden einzeln in Käfigen gehalten. Alle zwei Tage erhalten sie Kraftfutter. Wasser und Trockenfutter steht *ad libitum* zur Verfügung.

### APPLIKATION DER SUBSTANZEN

Um die genaue Dosierung der Substanzen ermitteln zu können, werden die Tiere am Abend vor der Substanzapplikation gewogen. Die oral zu verabreichenden Substanzen (Prodrugs) werden über eine Schlundsonde appliziert. Dazu werden das *O*-Succinylbenzamidoxim in 100 mM Phosphatpuffer pH 8,5 gelöst Das intravenös gegebene Benzamidin wird in NaCl-Lösung 0,9% gelöst, um eine Hämolyse zu verhindern. Nach der Injektion wird noch einmal mit mindestens 0,5 ml NaCl-Lösung 0,9% nachgespült Die Substanzapplikation erfolgt jeweils morgens.

Die Prodrugs werden drei Ratten appliziert. Benzamidin wird zwei Ratten intravenös appliziert Die oral verabreichten Dosen des *O*-Succinylbenzamidoxims betragen 50 mg/kg KG. Benzamidin wurde in einer Konzentration von 10 mg/kg KG appliziert.

### ENTNAHME DER BLUTPROBEN

Aus einer Ratte können sechs Blutproben gewonnen werden. Die Versuchsperiode für eine Kondition dauert einen Tag. Die Blutproben werden über einen Zeitraum von acht Stunden nach oraler Applikation bzw. sechs Stunden nach intravenöser Applikation gewonnen. Nach oraler Gabe erfolgt die Probenentnahme nach 30, 60, 90, 120, 240 und 480 Minuten, nach intravenöser Applikation nach 5, 10, 20, 40, 80 und 360 Minuten. Vor der Blutentnahme wird der Katheter durch kurzes Ansaugen bis zum Erscheinen von Blut geleert. Die Blutentnahme (300 µl) erfolgt mittels Multivetten (Multivetten^{®} 600, Sahrstedt, Nümbrecht). Zum Freihalten des Katheters werden nachträglich ca. 0,3 ml einer Mischung aus Heparin und NaCl gespritzt. Das gewonnene Vollblut wird zentrifugiert (1500 g, 10 min, 4°C). Nach der Zentrifugalion werden ca. 150 µl Plasma als Überstand abgenommen, in Eppendorfgefäße pipettiert und bei -80°C eingefroren.

### AUFARBEITUNG DER BLUTPROBEN

Nach langsamem Auftauen werden 150 µl Plasma mit *Aqua bidest.* ad 600 µl verdünnt. Anschließend werden die Plasmaproben mittels Festphasenextraktion aufgearbeitet Nach dem Konditionieren der Säule mit 1000 µl Methanol und dem Equilibrieren mit 1000 µl *Aqua bidest.* erfolgt der Probenauftrag (600 µl). Das Sorbens wird nach dem Probenauftrag mit 600 µl *Aqua bidest.* gewaschen. Die Elution der Substanzen erfolgt mittels *Aqua bidest.* pH 3/Methanol (6/4, V/V). Daraufhin wird das Eluat zur Trockne einkonzentriert und mit 100 µl *Aqua bidest.*/Methanol (9/1, V/V) aufgenommen und der HPLC zugeführt.

### HPLC-ANALYTIK ZUR TRENNUNG VON BENZAMIDOXIM UND BENZAMIDIN

Zur Trennung der zu analysierenden Substanzen wurde folgende HPLC-Methode verwendet

| | |
|---|---|
| HPLC-Pumpe: | Waters 600 |
| Detektor | Waters 2417 Tunable Absorbance Detektor |
| Autosampler: | Waters 717 plus Autosampler |
| Integrator: | EZChrom™ Elite Client/Server Version 2.8.3 Build 2249 Aufnahme und Auswertsoftware |
| Stationäre Phase: | Synergy Max-RP 80A; 250*4,6 mm mit Vorsäule C 18 4,0 * 3,0 mm (Phenomenex, Aschaffenburg) |
| Säulentemperatur: | konstant 24°C, mittels Säulenofen |
| Mobile Phase: | 10 mM Octylsulfonat in *Aqua bidest.,* pH 2,5 (mit konz. H₃PO₄)/Acetonitril (82,5/17,5, V/V) |
| Laufzeit: | 30 Minuten |
| Detektion: | UV-Detektor, 229 nm |
| Flussrate: | 1,0 ml/min |
| Injektionsvolumen: | 10 µl |
| Detektorempfindlichkeit: | Absorbance units fullscale: 2,000 |
| Retentionszeiten: | Benzamidoxim: 23,5 ± 0,5 min |
| | Benzamidin: 26,5 ± 0,5 min |

Der Eluent wurde mit einem Satorius Membranfilter (0,45 µm) filtriert und im Ultraschallbad 15 Minuten entgast.

### HPLC-ANALYTIK ZUR ANALYSE DES O-SUCCINYLBENZAMIDOXIMS, DES BENZAMIDOXIMS UND DES BENZAMIDINS

Zur Trennung der zu analysierenden Substanzen wurde folgende HPLC-Methode verwendet

| | |
|---|---|
| HPLC-Pumpe: | Waters 600 |
| Detektor: | Waters 2417 Tunable Absorbance Detektor |
| Autosampler: | Waters 717 plus Autosampler |
| Integrator: | EZChrom™Elite Client/Server Version 2.8.3 Build 2249 Aufnahme und Auswertsoftware |
| Stationäre Phase: | Synergy Max-RP 80A; 250 * 4,6 mm mit Vorsäule C 18 4,0 * 3,0 mm (Phenomenex, Aschaffenburg) |
| Mobile Phase: | 100 mM Phosphatpuffer in *Aqua bidest.,* pH 7,0 (mit 30% KOH)/Acetonitril (92/8, V/V) |
| Laufzeit: | 25 min |
| Detektion: | 229 nm |
| Flussrate: | 1,0 ml/min |
| Injektionsvolumen: | 10 µl |
| Detektorempfindlichkeit: | Absorbance units fullscale: 2,000 |
| Retentionszeiten: | Benzamidin: 5,3 ± 0,3 min |
| | *O*-Succinylbenzamidoxim: 11,7 ± 0,3 min |
| | Benzamidoxim: 15,2 ± 0,3 min |

Der Eluent wurde mit einem Sartorius Membranfilter (0,45 µm) filtriert und im Ultraschallbad 15 Minuten entgast

Aus den gewonnenen Daten konnte die orale Bioverfügbarkeit des Benzamidins nach oraler Gabe des *O*-Succinylbenzamidoxims bestimmt werden (Tab1):

**Tabelle 1: Bioverfügbarkeit des Benzamidins nach oraler Gabe des O-Succinylbenzaimdoxims**

| | Bioverfügbarkeit [%] | Mittelwert [%] | Staadardabweichuag [%] |
|---|---|---|---|
| Ratte 4 | 19,9 | | |
| Ratte 10 | 36,1 | 31,6 | 10,2 |
| Ratte 21 | 38,7 | | |

Wie obiger Tabelle zu entnehmen ist, besitzt Benzamidin nach oraler Gabe des *O*-Succinylbenzamidoxims eine Bioverfügbarkeit von 32%. Dies zeigt, dass das Prodrug nach oraler Gabe resorbiert und im Blut zur Wirkform reduziert wird.

Die Ergebnisse der Versuche sind in den beigefügten Zeichnungen dargelegt, in denen:

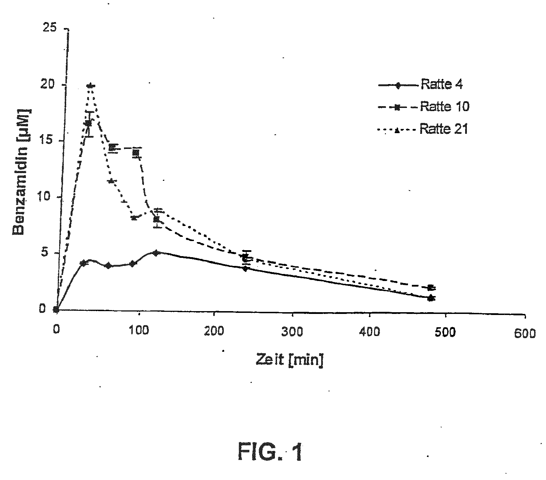

- Fig. 1: die Plasmaspiegelkurven des Benzamidins nach oraler Applikation des *O*-Succinylbenzamidoxims,
- Fig. 2: den Metabolismus des Diminazensuccinylsäureesters und
- Fig. 3: den Metabolismus des Pentamidinsuccinylsäureesters zeigt

### Besondere Ausführungsformen der Erfindung sind:

1. Verwendung eines Amidoximcarbonsäureesters der Formel (I) oder eines N-Hydxoxyguanidincarbonsäureesters der Formel (II) 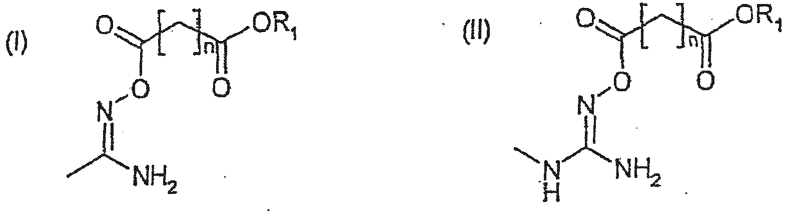 wobei n = 0,...,12 ist und R1 ausgewählt ist aus der Gruppe bestehend aus Wasserstoff, einem Alkylrest und einem Arylrest und deren Salze, als Ersatz einer oder mehrerer Amidin-, N-Hydroxyamidin- (Amidoxim), Guanidin- oder N-Hydroxyguanidin-Funktionen eines Arzneistoffs in Arzneimitteln zur Verbesserung der Löslichkeit, Bioverfügbarkeit und/oder Blut-Hirn-Schrankengängigkeit des Arzneistoffs.
2. Verwendung nach Ausführungsform 1,
   **dadurch gekennzeichnet,** dass
   der Arzneistoff ausgewählt ist aus der Gruppe der Proteasehemmstoffe, der DNA- und RNA-interkalierenden Verbindungen, der Hemmstoffe viraler Enzyme und der N-Methyl-D-Aspartat-Rezeptor-Antagonisten.
3. Verwendung nach Ausführungsform 2,
   **dadurch gekennzeichnet,** dass der Proteasehemmstoff ein Thrombinhemmstoff, ein Hemmstoff von Faktor Xa, Faktor VII oder aller Proteasen der Gerinnungskaskade oder ein Matriptasehemmstoff ist.
4. Verwendung nach Ausführungsform 2,
   **dadurch gekennzeichnet,** dass
   der Proteasehemmstoff ein Urokinasehemmstoff ist.
5. Verwendung nach Ausführungsform 2,
   **dadurch gekennzeichnet,** dass
   die DNA- und RNA-interkalierende Verbindung Pentamidin, Diminazen oder Isometamidium ist.
6. Verwendung nach Ausführungsform 2, **dadurch gekennzeichnet,** dass
   der Hemmstoff viraler Enzyme eine Neuraminidasehemmstoff ist.
7. Verwendung nach einem der vorgehenden Ausführungsformen,
   **dadurch gekennzeichnet,** dass
   der Arzneistoff zur Prophylaxe und Therapie der viszeralen und/oder kutanen Leishmaniose, der Trypanosomiasis, der 2. Phase der Trypanosomiasis oder der durch Pneumocystis carinii verursachten Pneumonie, zur Wachstumshemmung maligner Tumoren, zur Blutgerinnungshemmung, zur Blutdrucksenkung, zur Neuroprotektion oder zur Bekämpfung von viralen Infekten, einschließlich Influenza und HIV-Infektionen eingerichtet ist.

Besondere Ausführungsformen der Erfindung sind:
1. Verwendung eines Amidoximcarbonsäureesters der Formel (I) oder eines N-Hydroxyguanidincarbonsäureesters der Formel (II) 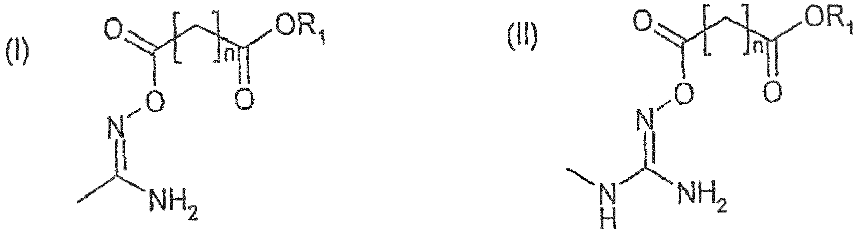 wobei n = 0,...,12 ist und R1 ausgewählt ist aus der Gruppe bestehend aus Wasserstoff, einem Alkylrest und einem Arylrest und deren Salze, als Ersatz einer oder mehrerer Amidin-, N-Hydroxyamidin- (Amidoxim), Guanidin- oder N-Hydroxyguanidin-Funktionen eines Arzneistoffs in Arzneimitteln zur Verbesserung der Löslichkeit, Bioverfügbarkeit und/oder Blut-Hirn-Schrankengängigkeit des Arzneistoffs,
   **dadurch** gekennzeichnet, dass
   der Arzneistoff ausgewählt ist aus der Gruppe der Proteasehemmstoffe, wobei der Proteasehemmstoff ein Thrombinhemmstoff, ein Hemmstoff von Faktor Xa, Faktor VII oder aller Proteasen der Gerinnungskaskade oder ein Matriptasehemmstoff ist, der DNA- und RNA-interkalierenden Verbindungen, der Hemmstoffe viraler Enzyme und der N-Methyl-D-Aspartat-Rezeptor-Antagonisten.
2. Verwendung nach Ausführungsform 1,
   **dadurch gekennzeichnet**, dass
   die DNA- und RNA-interkalierende Verbindung Pentamidin, Diminazen oder Isometamidium ist.
3. Verwendung nach Ausführungsform 1,
   **dadurch gekennzeichnet**, dass
   der Hemmstoff viraler Enzyme eine Neuraminidasehemmstoff ist.
4. Verwendung nach einer der vorgehenden Ausführungsformen,
   **dadurch gekennze ichnet,** dass
   der Arzneistoff zur Prophylaxe und Therapie der viszeralen und/oder kutanen Leishmaniose, der Trypanosomiasis, der 2. Phase der Trypanosomiasis oder der durch Pneumocystis carinii verursachten Pneumonie, zur Wachstumshemmung maligner Tumoren, zur Blutgerinnungshemmung, zur Blutdrucksenkung, zur Neuroprotektion oder zur Bekämpfung von viralen Infekten, einschließlich Influenza und HIV-Infektionen eingerichtet ist.

## Patentansprüche

1. Verwendung eines Amidoximcarbonsäureesters der Formel (I) oder eines N-Hydroxyguanidincarbonsäureesters der Formel (II) 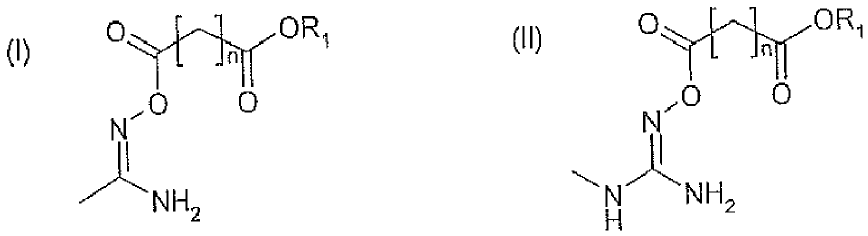 wobei n = 0,...,12 ist und R1 ausgewählt ist aus der Gruppe bestehend aus Wasserstoff, einem Alkylrest und einem Arylrest und deren Salze, als Ersatz einer oder mehrerer Amidin-, N-Hydroxyamidin- (Amidoxim), Guanidin- oder N-Hydroxyguanidin-Funktionen eines Arzneistoffs in Arzneimitteln zur Verbesserung der Löslichkeit, Bioverfügbarkeit und/oder Blut-Hirn-Schrankengängigkeit des Arzneistoffs.

2. Verwendung nach Anspruch 1,
**dadurch gekennzeichnet, dass**
der Arzneistoff ausgewählt ist aus der Gruppe der Proteasehemmstoffe, wobei der Proteasehemmstoff ein Thrombinhemmstoff, ein Hemmstoff von Faktor Xa, Faktor VII oder aller Proteasen der Gerinnungskaskade oder ein Matriptasehemmstoff ist, der DNA- und RNA-interkalierenden Verbindungen, der Hemmstoffe viraler Enzyme und der N-Methyl-D-Aspartat-Rezeptor-Antagonisten

3. Verwendung nach Anspruch 2,
**dadurch gekennzeichnet, dass**
die DNA- und RNA-interkalierende Verbindung Pentamidin, Diminazen oder Isometamidium ist.

4. Verwendung nach Anspruch 2,
**dadurch gekennzeichnet, dass**
der Hemmstoff viraler Enzyme eine Neuraminidasehemmstoff ist.

5. Verwendung nach einer der vorgehenden Ansprüche,
**dadurch gekennzeichne**t, dass
der Arzneistoff zur Prophylaxe und Therapie der viszeralen und/oder kutanen Leishmaniose, der Trypanosomiasis, der 2. Phase der Trypanosomiasis oder der durch Pneumocystis carinii verursachten Pneumonie, zur Wachstumshemmung maligner Tumoren, zur Blutgerinnungshemmung, zur Blutdrucksenkung, zur Neuroprotektion oder zur Bekämpfung von viralen Infekten, einschließlich Influenza und HIV-Infektionen eingerichtet ist.

6. Prodrug umfassend eine die Formel (I) oder (II) aufweisende Teilstruktur, 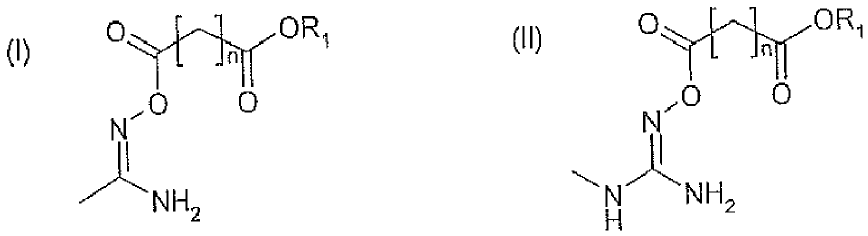 wobei n = 0,...,12 ist und R1 ausgewählt ist aus der Gruppe bestehend aus Wasserstoff, einem Alkylrest und einem Arylrest, welches ein Prodrug zu einem Arzneistoff ist.

7. Prodrug gemäß Anspruch 6,
**dadurch gekennzeichnet, dass** der Arzneistoff ausgewählt ist aus der Gruppe der Proteasehemmstoffe, wobei der Proteasehemmstoff ein Thrombinhemmstoff, ein Hemmstoff von Faktor Xa, Faktor VII oder aller Proteasen der Gerinnungskaskade oder ein Matriptasehemmstoff ist, der DNA- und RNA-interkalierenden Verbindungen, der Hemmstoffe viraler Enzyme und der N-Methyl-D-Aspartat-Rezeptor-Antagonisten.

8. Prodrug gemäß Anspruch 7,
**dadurch gekennzeichnet, dass** die DNA- und RNA-interkalierende Verbindung Pentamidin, Diminazen oder Isometamidium ist.

9. Prodrug gemäß Anspruch 7,
**dadurch gekennzeichnet, dass** der Hemmstoff viraler Enzyme eine Neuraminidasehemmstoff ist.

10. Prodrug gemäß mindestens einem der Anprüche 6-9,
**dadurch gekennzeichnet, dass** der Arzneistoff zur Prophylaxe und Therapie der viszeralen und/oder kutanen Leishmaniose, der Trypanosomiasis, der 2. Phase der Trypanosomiasis oder der durch Pneumocystis carinii verursachten Pneumonie, zur Wachstumshemmung maligner Tumoren, zur Blutgerinnungshemmung, zur Blutdrucksenkung, zur Neuroprotektion oder zur Bekämpfung von viralen Infekten, einschließlich Influenza und HIV-Infektionen eingerichtet ist.

11. Prodrug gemäß mindestens einem der Ansprüche 6-10,
wobei die Gesamtstruktur des Prodrugs mehrere Teilstrukturen der Formel (I) und/oder (II) umfasst.
